# EUROPEAN PATENT APPLICATION

(11) **EP 0 530 429 A1**
(43) Date of publication of application: **10.03.1993**
(21) Application number: 91830361.1
(22) Date of filing: 30.08.1991
(51) Int. Cl.: A61B 17/32, A61C 15/04

(54) **An improved multi-purpose cartridge type handle assembly and its sanitary accessories**

(71) Applicant: Lai, Ming-Der, Pan Chiao City, Taipei Hsien (TW)
(72) Inventor: Lai, Ming-Der, Pan Chiao City, Taipei Hsien (TW)
(74) Representative: Righetti, Giuseppe

(57) **Abstract**

The present disclosure is related to an improved multi-purpose cartridge type handle assembly and its sanitary accessories, which is characterized in the rotary knob adjustor (6b) disposed at the end of the handle assembly so that a movable urging block (2) wedged in a U-shaped bracket (11) can be released and tightened as a result of the activation of the knob adjustor (6b) in either clockwise or counter-clockwise direction, permitting the easy replacement of selected sanitary accessory in need by attachment of an adaptor disposed at the front of the handle assembly. Furthermore, a dental floss adaptor (13) can be easily disassembled for replacement of dental floss and repeatedly used; and this adaptor can be turned around in 360 degrees.

## Description

The present invention is related to an improved multi-purpose cartridge type handle assembly onto which a number of sanitary accessories can be replaceably attached, and in particular, to a handle body to the end of which is attached a threaded bar section, and the section is rotatably passed through a handle head and terminated in a knob adjustor. By turning of the knob adjuster, the threaded bar section is able to make the handle head to move forward or backward along with a U-shaped bracket to which is slidably secured a movable urge block. The block is provided with a tongue member at the front end thereof, which will press against the front portion of the U-shaped bracket so to permit the adaptor of sanitary accessory selected to be held tightly in place or to be detached from the front end of the handle body.

The present inventor has come up with a first generation multi-purpose cartridge type handle assembly, U.S. patent No. 4,942,894, which is provided with a spring-urged movable block. The front end of the movable block is equipped with a tongue against which a selected adaptor can be held in place as a result of the exertion of a spring disposed between the end of the block and the end of the handle body. The movable block is slidably wedged in a U-shaped bracket with the spring urged against the same. By pushing the movable block backward against the spring, the adaptor to which a kind of sanitary accessory is attached can be removed from the handle assembly.

However, the prior handle assembly is not satisfactory in operation because of the spring element being easily in fatigue after a long period of time, and the adaptor attached at the front end of the handle assembly will get loose in use easily.

Moreover, the specific adaptor for attaching dental floss in the prior art of the inventor is made integrated in structure with the floss not being able to be replaced. The present inventor provides an improved adaptor which can be assembled and disassembled, permitting a selected dental floss to be mounted thereto with ease so that the adaptor can be reused as many times as one wishes without disposal.

The primary object of the present invention is to provide a rotatable knob adjustor for a multi-purpose handle assembly in replacement of a prior spring activated urging block. By rotation of the knob adjustor in either direction, the movable urging block will be forced to move forward or backward so that the detachable sanitary adaptor urged by the urging block at the frontmost end of the handle body can be either removed or held tightly in place without loosening.

One further object of the present invention is to provide a multi-purpose handle assembly to which a dental floss adaptor can be removably mounted; and the dental floss thereon can be easily replaced when needed so that a person can choose the proper type of floss as wishes.

To better illustrate the structure, operation modes and features of the present invention a number of drawings in company with a detailed description of the preferred embodiment are given, in which:
- Fig. 1 is a diagram showing the perspective exploded sanitary accessories attached to the cartridge type handle assembly;
- Fig. 2 is a diagram showing the separated parts of the present handle assembly;

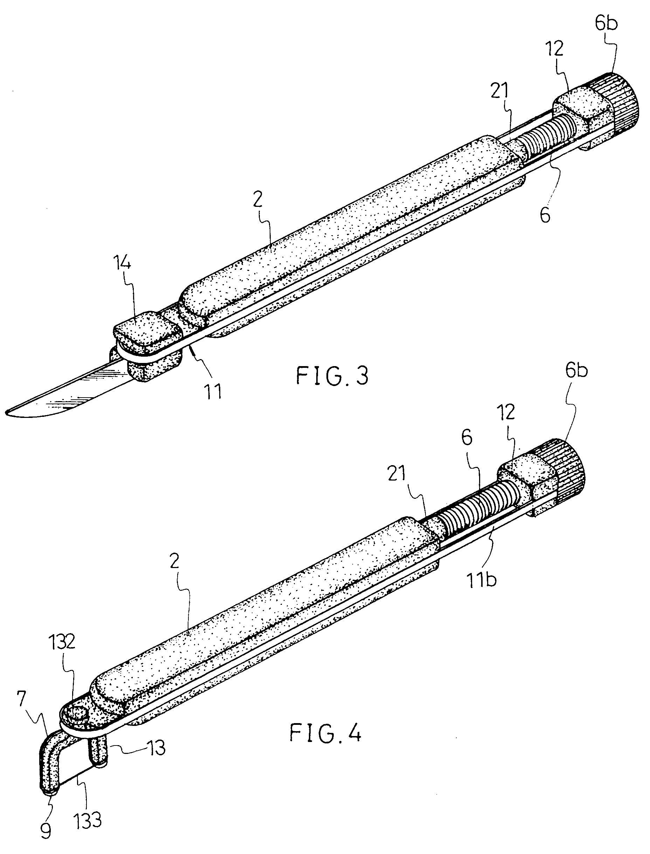

- Fig. 3 is a diagram showing the attachment of one type of sanitary accessory to the handle assembly of the present invention;
- Fig. 4 is a diagram showing the attachment of other type of sanitary accessory to the handle assembly of the present invention;
- Fig. 5 is a sectional view showing the rotatable knob type adjustor of the present invention;
- Fig. 6 is a diagram showing the floss-replaceable dental floss adaptor of the present invention;

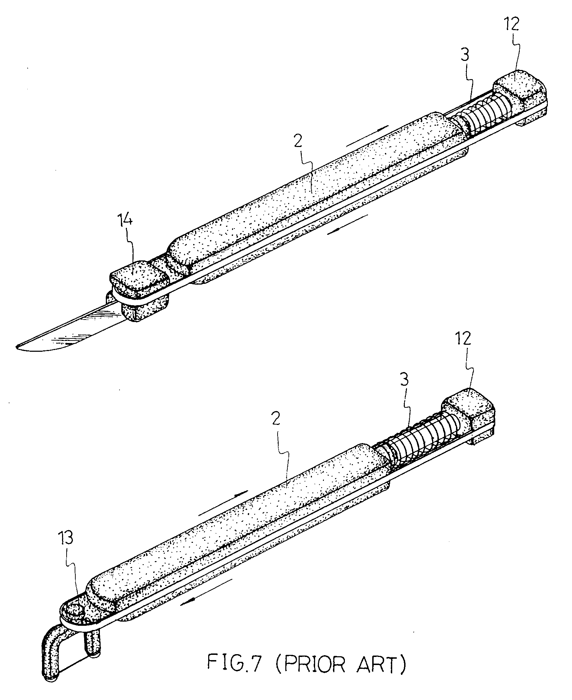

- Fig. 7 is a diagram showing the spring urged structure of the prior art.

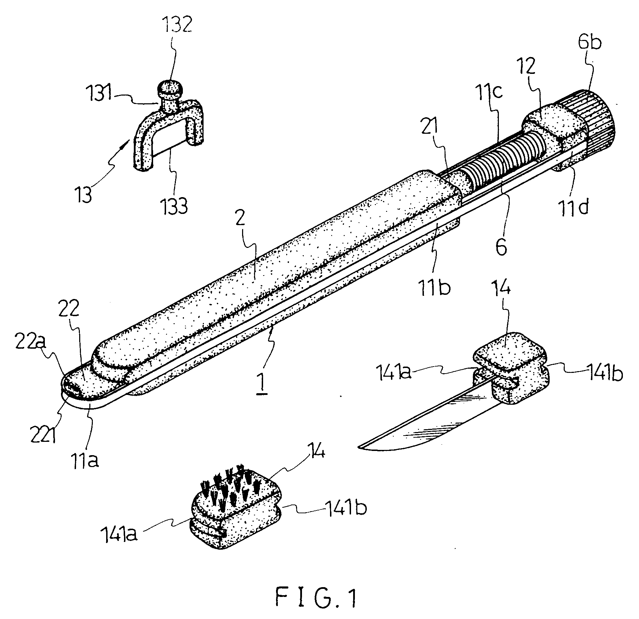

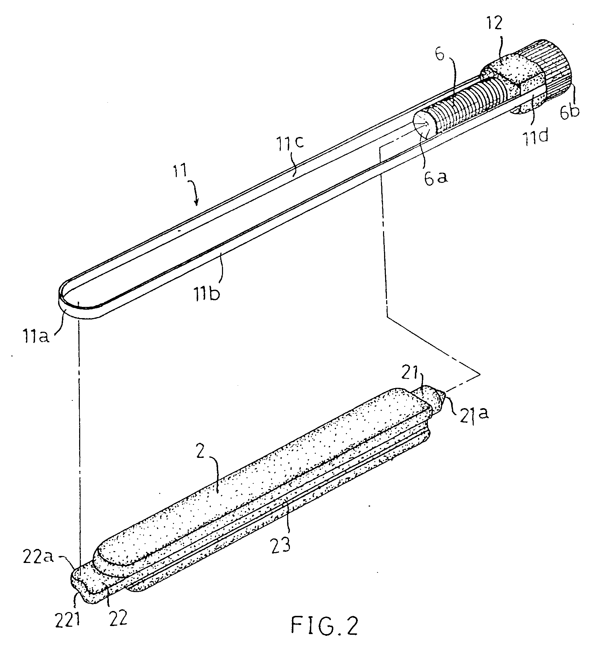

Referring to Figs. 1, 2, the present multi-purpose cartridge type handle assembly is comprised of a handle body 1, a dental floss adaptor 13 and a tooth brush adaptor 14 and an operation knife adaptor 14.

The handle body 1 has a hand hold movable block 2 which is provided with a protrusion tongue 22 at the front end thereof. At the opposite end of the block 2 is disposed a round tenon 21. On the sides of the movable block 2 are defined concave slots 23 for the mounting of a U-shaped bracket 11 having continuously connected portions 11a, 11b, 11c, 11d onto the block 2. The front portion 11a is in contact with the protrusion tongue 22, and the portions 11b, 11c are fit in the side concave slots 23 along the periphery of the movable block 2, and the portion lid is engaged with a handle head 12.

At the end of the handle body 1 is disposed a threaded bar section 6 which is rotatably passed through the handle head 12. At the frontmost end of the threaded bar section is defined a recess 6a so that the pointed protrusion 21a of the round tenon 21 can be fit therein. A knob adjustor 6b is integrally connected to the threaded bar section 6; turning the knob adjustor 6b will permit the bar section 6 to rotate so that the U-shaped bracket 11 along with the handle head 12 will be activated to move forward or backward accordingly.

The movable block 2 engaged with the threaded bar section 6 by way of the pointed protrusion 21a of the round tenon 21 will be activated to move forward when the knob adjustor 6b is turned in one specific direction so to make the adapter 13 or 14 to be held tightly in place by the tongue 22. A bow shaped edge 221 on the front end 22a of the tongue 22 is defined to permit the easy holding of the adaptor. When the knob adjustor is turned in the other direction, the threaded bar section 6 will move backward so that the movable block 2 is able to slide with respect to the U-shaped bracket 11 with a space produced between the tongue 22 and the front portion 11a of the bracket 11. Thereby the held adaptor can be taken from the front end of the handle assembly for replacement of sanitary accessory.

In detail, the sanitary accessories attached to the adaptors 13, 14 can be selectively mounted onto the handle assembly by simply turning the knob adjustor in one direction to produce a space between the tongue 22 and the front portion 11a of the bracket 11, as described in the proceedings, so that the adaptor 14 having a front concave slot 141a and a rear slot 141b can be inserted into the space between the tongue 22 and the portion 11a with the bow shaped edge 221 on the front end 22a of the tongue 22 engaged with the rear slot 141b of the adapter 14 and the front slot 141a in engagement with the front portion 11a; afterwards, the knob adjustor 6b is turned in the opposite direction so to urge the movable block move against the U-shaped bracket, holding the adapter tightly in position. To detach the secured adapter, one only has to turn the knob adjuster in a reverse direction.

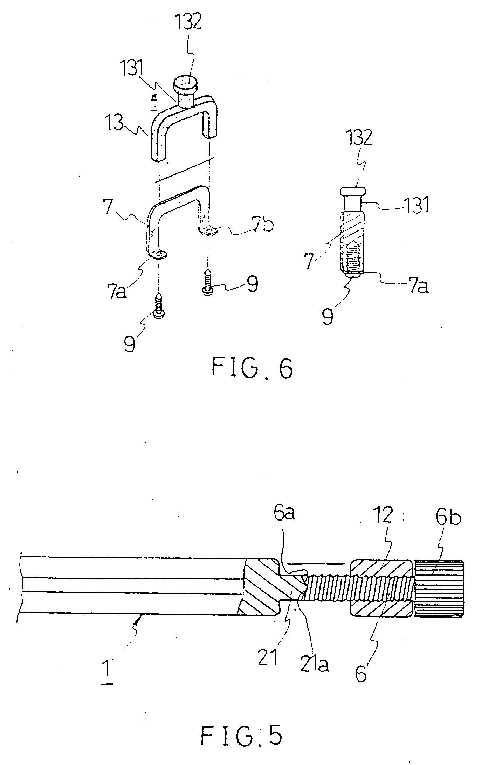

The dental floss adapter 13 has a U-shaped main member at the top of which is disposed a vertical neck 131 with a disk-like unit 132 extended from the top thereof. A U-shaped attachment member 7 having two horizontal bends 7a, 7b at the bottom of its two legs respectively. A through hole is disposed on each bend 7a or 7b. Each leg of the main member is disposed a drill so that a screw 9 can be used to hold the main member and the attachment member together as shown in Fig. 6. A section of dental floss can be fixed between the main member and the attachment member first and the screws 9 are used to fix the two elements together then, so to hold the dental floss 133 firmly in place. To mount the dental floss adaptor 13 to the handle assembly, the neck 131 is disposed in the space between the front portion 11a of the bracket 11 and the tongue 22. The neck 131 is held tightly in place by the bow shaped edge 221 on the front end 22a of the tongue 22. Therefore, the dental floss 133 can be easily replaced and the adaptor can be turned around in 360 degrees.

It becomes apparent that the present invention can be operated in better and more efficient manner with the dental floss on the dental floss adaptor easily replaced, and the knob adjustor and the threaded bar section permit the attachment and detachment of the adaptors with ease.

## Claims

1. An improved multi-purpose cartridge type handle assembly mainly comprising a handle body (1) having a movable urge block (2) and a U-shaped bracket (11); and a number of sanitary adaptor means to which sanitary accessories, such as dental floss tooth brush and operation knife can be secured; said movable block (2) having a protrusion tongue (22) at one end thereof which is provided with a bow shaped edge (221) at the front end of said protrusion tongue (22); a round tenon (21) being disposed at the end of said movable block (2), opposite to the end of said protrusion tongue (22); said round tenon (21) terminating in a pointed protrusion (21a); along the continuous periphery of said movable block (2) being defined concave slots (23); the U-shaped bracket (11) being able to be slidably engaged with said movable block (2) by way of said concave slots (23); a handle head (12) being disposed at the end of said U-shaped bracket (11); and a threaded bar section (6) being rotatably disposed through said handle head (12) and a recess (6a) to be defined at the end thereof; said recess being in engagement with said pointed protrusion (21a) of said round tenon (21); and a knob adjustor (6b) being secured at the other end of said threaded bar section (6); by turning said knob adjustor (6b) in one direction the threaded bar section (6) being able to make said handle head (12) move along with said U-shaped bracket (11) with respect to said movable urge block (2) so that a space being produced between the front portion (11a) of said bracket (11) and said tongue (22) of said movable block (2), permitting said adaptor means to be inserted in said space and held tightly in place when said knob adjustor (6b) is turned in opposite direction.

2. An improved multi-purpose cartridge type handle assembly as claimed in claim 1, wherein said dental floss adaptor means is equipped with a U-shaped main member (13) having two legs each of which has a drill at the bottom thereof; and also with a U-shaped attachment member (7) having two legs the bottom ends thereof being horizontally bent with a through hole defined thereon respectively so that said main member (13) can be securely joined to said attachment member (7) by a pair of screws (9) when a section of dental floss is disposed between said legs of said main member and said attachment member for use.

3. An improved handle assembly as claimed in claim 1 wherein said dental floss adaptor means is provided with a vertical neck (131) at the top of said main member, which terminates in a disk-like unit (132); thereby said neck can be placed between the bow shaped edge (221) of said tongue (22) and the front portion (11a) of said U-shaped bracket (11) and held tightly in position as long as said movable block (2) is actuated to move against said bracket (11); and said tooth string adaptor can be turned around 360 degrees for proper use.
